**Europäisches Patentamt**

**European Patent Office**

**Office européen des brevets**

(19)

(11) Publication number : **0 486 399 A1**

## (12) EUROPEAN PATENT APPLICATION

(21) Application number : **91403064.8**

(22) Date of filing : **14.11.91**

(51) Int. Cl.⁵ : **A61B 5/04**

(30) Priority : **14.11.90 CU 17790**

(43) Date of publication of application :
**20.05.92 Bulletin 92/21**

(84) Designated Contracting States :
**AT BE CH DE DK ES FR GB GR IT LI LU NL SE**

(71) Applicant : **CENTRO DE NEUROCIENCIAS DE CUBA**
**Calle 25 No. 15202, esq. 158, Cubanacan, Playa**
**La Habana 12100 (CU)**

(72) Inventor : **Sanchez Castillo, Manuel**
**Ave. 23A no. 23016, la Lisa**
**La Habana (CU)**

(74) Representative : **Joly, Jean-Jacques et al**
**CABINET BEAU DE LOMENIE 55, rue d'Amsterdam**
**F-75008 Paris (FR)**

(54) **Biopotential amplifier.**

(57) The invention refers to a generic, digitally controlled bioelectric signal amplifier for use in computerized electrophysiological systems. It offers a solution for a broad range of adjustment of high and low pass filtering with circuits that occupy a small volume. In case of fixed gain amplifiers, such as those used for ECG and EEG, a simple solution is proposed for the reduction of recovery time after an out of range input. The amplifier comprises four blocks : 10, 20, 30 ,40. Original solutions are included in the design of blocks 20 and 40. Block (20) is a high pass filter that with the next amplifier stage contains a feedback circuit. This close loop multiplies the RC time constant by a coefficient of a value greater than 1 for input levels inside the range and less than 1 for out of range input levels. The last block (40) is formed by a sweepable switched capacitor low pass filter. Block (10) is a pre-amplifier and block (30) is a third amplification stage.

**EP 0 486 399 A1**

Fig. 1

The present invention relates to the field of electronic medical equipment and more specifically that of amplifiers for bioelectrical signals such as Electroencephalogram (EEG), Cerebral Evoked Potentials (EP), or Electrocardiogram (ECG). In the context of multichannel computerized systems the amplifiers function as a software controlled signal conditioning block.

These signals are recorded in several frequency bands and have an added higher voltage continuous component, essentially due to the polarization potential of the electrodes. Thus, high pass-filters are needed before the total amplification of the signal. Also an automatic reduction of RC time constant in the presence of a voltage overload at the amplifier input has to be considered in order to avoid a prolonged blockage of the signal (particularly for high time constants). Finally low-pass antialiasing filter with different cut-off frequencies are needed.

Existing solutions occupy a considerable amount of space on printed circuit cards to perform these functions. This is due, to the number of capacitors needed for the implementation of the several cut-off frequencies, as well as the size of the value capacitors which are needed to obtain the larger time constants in the high pass filter. Additionally, the solutions for the time constant automatic reduction,as in the case of the Rumanian patent No.77897 , not only uses a relatively great amount of components, such as logic gates, but interrupts transitorially the signal during a time after the overvoltage aparition, in order to obtain the complete capacitor discharge. This solution can contribute notably to the signal acquisition process delay in such cases where the presence of overvoltages generating artifacts is quite often.

The objectives of the proposed solution are:

To reduce the number and capacity of the capacitors as well as the number of analog switches used for the implementation of different cut-off frequencies in the high pass filter; by means of simpler and faster operating circuit in the high-cut filter to reduce the recovery time of the EKG or EEG amplifier when the signal is blocked following an input overload;

To improve the performance/component-count ratio in the low pass filters of amplifiers requiring several cut-off frequencies.

The RC high pass filter of the proposed generic amplifier, icludes a positive feedback circuit which acts as, an RC time constant multiplier when is activated by means of analog switch. Thus, it is possible to duplicate the number of cut-off frequencies steps, e.g. from 4 steps obtained by switching of a resistance and a capacitor, up to 8 steps using only 4 analog switches, without requiring high-value capacitors or resistances to obtain higher time constants (3 seconds or more).

In fixed gain amplifiers (such as those used for EEG and ECG recordings) another feedback loop is added through two avalanche which conduce when an overvoltage refered to the analog conversor input range occurs at the amplifier output. Due th the established negative feedback, this solution allows, under the above mentioned condition, to reduces the nominal time constant to a lower value. It also makes posible a fast recovery of the output level voltage down to a value close to the maximum of the operating range which results in a significantly lower signal blocking time. The proposed low pass filter is clock sweepable switched capacitor filter contained in a 14 pin IC such as LTC-1064-3 from LINEAR TECHNOLOGY.

A more detailed description of the invention is contained in the following annexed figures;

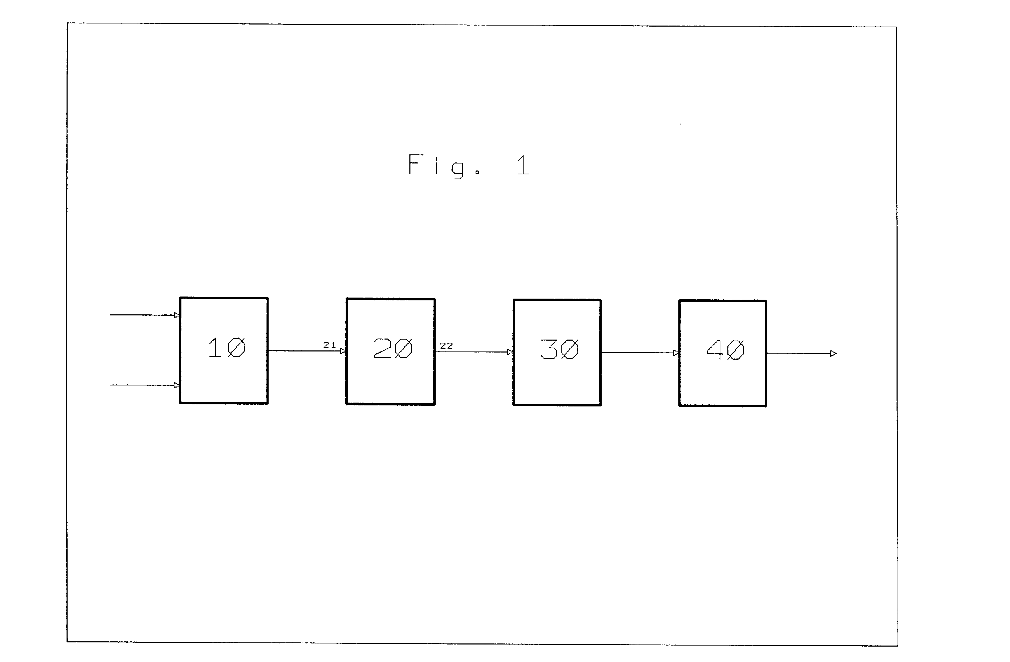

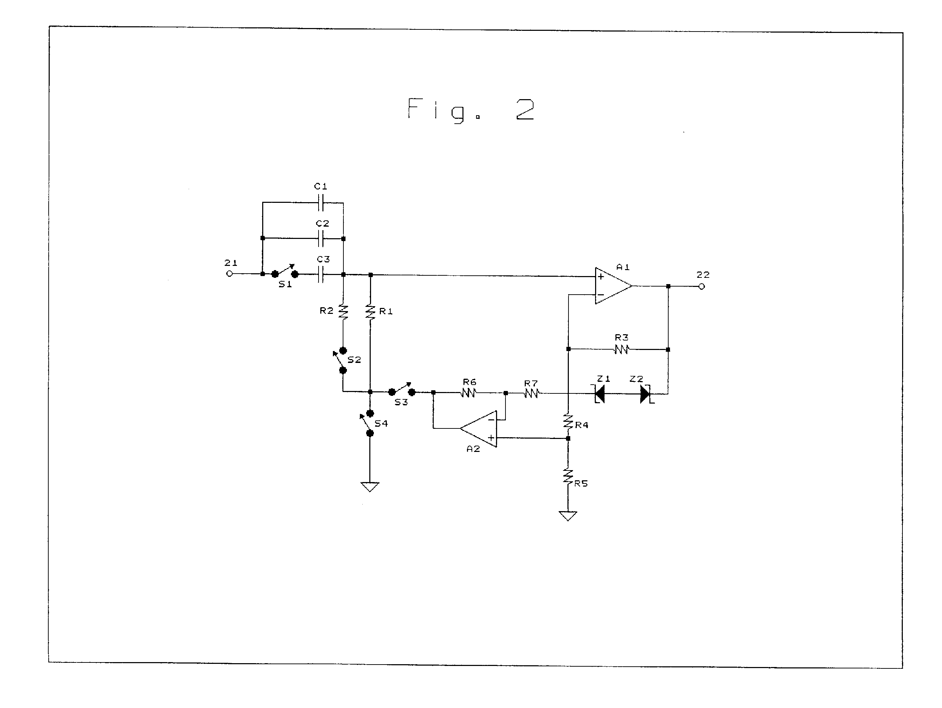

Figure 1 is a block diagram of an amplifier builded according to the proposed invention. Figure 2 is a detailed schematic representation of block No. 20, corresponding to the high pass filter circuit and the second amplification stage.

The amplifier, as presented in Figure 1, comprises a pre-amplifier (10) connected to a low pass RC filter, a second amplification stage (20) whose output is connected to an optical isolation and and a third amplification stage (30). The output of this stage is in turn, connected to a clock sweepable switched capacitor low pass filter.

The output of the pre-amplifier (10) is connected to the input (21) of the high pass filter and to the second amplification stage (20) illustrated in figure 2 and described is follows.

C1 connected in parallel to C2 and to the branch formed by the series connection of S1 and C3 forms the capacity C of the RC filter, connected at one end to the input (21) and at the other end with the junction of R1, R2 and the noninverting input of A1. Resistance R1 connected in parallel to the branch formed by the series connection of R2 and S2, is connected through S3 with the output of A2 or with ground (0 V) via S4. A1 forms a non inverting amplifier. R3 is connected between the output and the inverting input and the branch formed by the series connection of R4 and R5 is connected between the inverting input and ground (0 V). The junction of R4 and R5 is connected to the non-inverting input of A2, whose output is connected to S3 and R3. The branch formed by the series connection of R7, Z1 and Z2 is connected between the inverting input of $A_2$ (also connected to R6) and the output of A1, which is the output (22) of block 20.

The transfer function $H(s) = V_{22}(s)/V_{21}(s)$ of the circuit, where $V_{21}(s)$ is the voltage at input (21) and $V_{22}(s)$ is the voltage at output (22), is as follows

$$H(s) = G_2 \left[ \frac{\tau s}{\tau s + 1} \right]$$

where $G_3 = \dfrac{R_3}{R_4 + R_5} + 1$

$$\tau = \begin{cases} RC, & S_3 \text{ open and } S_4 \text{ closed.} \\ \left[ \dfrac{R_5}{R_4} + 1 \right] RC, & S_3 \text{ closed, } S_4 \text{ open, and } |V_{22}| < \left[ 1 + \dfrac{R_5}{R_3 + R_4} \right] (V_Z + V_F), \end{cases}$$

$$R = \begin{cases} R_1, & S_2 \text{ open} \\ R_1 || R_2, & S_2 \text{ closed} \end{cases}$$

$$C = \begin{cases} C_1 + C_2, & S_2 \text{ open} \\ C_1 + C_2 + C_3, & S_1 \text{ closed} \end{cases}$$

and $V_Z$, $V_F$ are the Zener or avalanche voltage, and the forward conduction voltage of the $Z_1$ and $Z_2$ diodes respectively.

When $S_3$ is closed and S4 is open, the positive feedback provided by $A_2$ -which acts as a voltage follower under the condition

$$|V_{22}| < \left[ 1 + \frac{R_5}{R_3 + R_4} \right] (V_Z + V_F) .$$

-provides the means to multiply the RC time constant avoiding the need for high-value capacitors or resistances to obtain higher time constants, e.g., equal to or over 3 sec., corresponding to a cut-off frequency of 0.05Hz and allowing also for the multiplication -- through the action of S3 and S4 -- of the number of steps for the adjustment of obtained through the change of value of C and R by the action of S1 and S2. Thus, for example, assigning the following relative values for C1, C2, C3, R1, R2, R4 and R4: C2 = 10C1; C3 = 10C2; R2 = R1; R5/R4 = 100, the time constant, $\tau$, of the filter may be adjusted in two steps with a 2:1 ratio by S2; in two steps with a 10:1 ratio by S1 and in two steps with a 100:1 ratio by S3 and S4, thus allowing for a total of 8 steps.

The branch formed by the series connection of Z1, Z2 and R7 between the output of A1 (22) and the inverting input of A2, and the resistance R6 between the said inverting input and the output of A2, determine that for

$$|V_{22}| > \left[ 1 + \frac{R_5}{R_3 + R_4} \right] (V_Z + V_F);$$

S3 closed, and S4 open the following holds:

$$\tau = \frac{RC}{\left[ \dfrac{R_6}{R_7}(R_3 + R_4) - R_5 \right] \dfrac{1}{R_5 + R_4} + 1}$$

which implies that $\tau < RC$, if R3 > R5 and R6$\cong$ R7.

Thus, with G34 being the gain of blocks 30 and 40, Vm the maximum or full scale voltage of the A/D converter, and selecting the values of G34 and Vz such that

$$G_{34} \left[ 1 + \frac{R_5}{R_3 + R_4} \right] (V_Z + V_F) = V_m$$

the time constant, $\tau$, assumes a value much lower than the nominal if the voltage at the output of the amplifier exceeds the operating range of the system when using the higher time constants through the effect of the feedback provided by A2, which under these conditions becomes negative.

When a voltage overload is present at the input of the amplifier, the time that the signal remains blocked is comparable to the lower time constants and we get a faster recovery to a within range level near the maximum.

The output (22) of block 20 is connected to the input (31) of an isolating block (30), whose output (32) is connected to the output (41) of the low-pass filter and the final buffer amplifier represented by block 40.

For biopotential amplifiers of general purposes , this invention proposes the use of a clock sweepable switched-capacitor low pass filter IC such as LTC1064-3 manufactured by Linear Technology which is, furthermore, of the 8-pole Bessel type.

This is a minimum space consuming solution to obtain adjustable cut-off frequencies in multiple steps within a broad range, as well as an excellent frequency response.

## Claims

1. Biopotential amplifier characterized by having a pre-amp (10) connected in series to a high-pass RC filter with digitally controlled cut-off frequencies associated to a second amplification stage (20) whose output is connected to an isolation stage and to a third amplification stage (30) connected in series with a low-pass filter and the output buffer amplifier (40)

2. A biopotential amplifier according to claim No.1 with a first order high pass RC filter whose capacity is formed by the parallel connection of C1, C2 and the branch formed by the series connection of S1 and C3 whose resistance R is formed by the parallel connection of R1 with the branch formed by the series connection of R2 and S2, characterized by having a feedback circuit that multiplies the RC time constant in a loop with the following amplification stage (A1), such that, A1 being an operational amplifier in a non inverting configuration with the junction of R and C (junction of C1, C2, C3, R1 and R2) connected to the non-inverting input, R3 connected between the output and the inverting input and the branch formed by the series connection of R4 and R5 connected between the inverting input and ground (0 V). The junction of R4 and R5 is connected to the non-inverting input of a second operational amplifier (A2). The inverting input of A2 is connected to its output through R6 just forming a voltage follower. The other end of the resistance R (junction of R1 and S2) of the RC filter may be connected to the output of A2 via S3 or to ground via S4.

3. A Biopotential amplifier according to claims No.1 and No.2 characterized by the fact that the described high pass filter feedback circuit has an additional loop through the branch formed by the series connection of R7, Z1 and Z2 between the output of A1 and the inverting input of A2 which in connection with R6 forms an inverting amplifier.

4. Biopotential amplifier according to claim No.1 characterized by the fact that the low pass filter (40) is a clock sweepable switched capacitor low pass filter.

Fig. 1

Fig. 2

European Patent
Office

**EUROPEAN SEARCH REPORT**

Application Number

EP    91 40 3064

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | EP-A-0 079 009 (HONEYWELL AND PHILIPS MEDICAL ELECTRONICS B.V.)<br>* abstract; figure 3 *<br>* page 4, line 5 - line 23 *<br>--- | 1-3 | A61B5/04 |
| A | EP-A-0 375 106 (PIONEER ELECTRONIC CORPORATION)<br>* abstract; figure 4 *<br>* column 9, line 23 - line 30 *<br>----- | 4 | |
| | | | **TECHNICAL FIELDS SEARCHED (Int. Cl.5 )**<br><br>A61B<br>H03F<br>H03H |

The present search report has been drawn up for all claims

| Place of search | Date of completion of the search | Examiner |
|---|---|---|
| BERLIN | 19 FEBRUARY 1992 | DANIELIDIS S. |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

&  : member of the same patent family, corresponding
document

EPO FORM 1503 03.82 (P0401)